# EUROPÄISCHE PATENTSCHRIFT

(11) **EP 2 552 198 B1**
(45) Veröffentlichungstag und Bekanntmachung des Hinweises auf die Patenterteilung: **14.09.2016**
(21) Anmeldenummer: 11709963.0
(22) Anmeldetag: 25.03.2011
(51) Int. Cl.: A01N 25/32, A01N 25/34, A01N 47/44, A61L 15/22, A61L 15/42, A61K 9/00, A61L 15/26, A61L 15/44, A61L 15/46, A61K 31/155

(54) **ANTIMIKROBIELLE WUNDAUFLAGE**
ANTIMICROBIAL WOUND DRESSING
PANSEMENT ANTIMICROBIEN

(30) Priorität: 26.03.2010 DE 102010013075
(43) Veröffentlichungstag der Anmeldung: 06.02.2013
(73) Patentinhaber: B. Braun Melsungen AG, 34212 Melsungen (DE)
(72) Erfinder: ARNDT, Andreas, 6010 Kriens (CH); PREUSS, Andrea, 4600 Olten (CH); SCHMITT, Jürgen, 35274 Kirchhain (DE); SIPPEL, Martin, 34212 Melsungen (DE); RIEMANN, Thomas, 37247 Großalmerode (DE); WEIß, André, 34302 Guxhagen (DE); KRAMER, Axel, 17489 Greifswald (DE); MÜLLER, Gerald, 17498 Hinrichshagen (DE)
(74) Vertreter: dompatent von Kreisler Selting Werner - Partnerschaft von Patent- und Rechtsanwälten mbB
(86) Internationale Anmeldenummer: PCT/EP2011/054617
(87) Internationale Veröffentlichungsnummer: WO 2011/117384

(56) Entgegenhaltungen:
- WO-A1-00/65915
- WO-A2-2007/089763
- US-A1- 2006 110 370

## Beschreibung

Die Erfindung betrifft eine Wundauflage umfassend ein polymeres Substrat und eine Zusammensetzung umfassend mindestens einen antimikrobiellen Wirkstoff sowie ein die Zytotoxizität absenkendes Mittel. Darüber hinaus betrifft die Erfindung die Herstellung einer solchen Wundauflage.

Antimikrobielle Wirkstoffe werden in unterschiedlichen kosmetischen und medizinischen Produkten sowohl zur Konservierung des jeweiligen Produkts als auch zur Erzielung einer antimikrobiellen Wirkung auf der Haut oder Schleimhaut, d.h. zur Desinfektion, Antisepsis und Reduktion eines Infektionsrisikos verwendet. Dabei weisen antimikrobielle Wirkstoffe aufgrund ihrer beabsichtigten biologischen Wirkung immer auch ein gewisses Risiko der Schädigung menschlicher Zellen auf. Insbesondere ist es erwünscht, dass solche Wirkstoffe einen unspezifischen Wirkungsmechanismus zeigen, um der eventuellen Entstehung von Resistenzen oder gar Kreuzresistenzen gegen Antibiotika vorzubeugen.

Auf der anderen Seite sollen Produkte, die solche Wirkstoffe enthalten, keine schädigende Wirkung auf menschliches Gewebe haben. Dies ist von noch größerer Bedeutung, wenn solche Produkte in Kontakt nicht nur mit gesunder Haut kommen, sondern insbesondere bei Produkten, die in der Wundbehandlung eingesetzt werden.

Polymere Guanidine sowie polymere Biguanide, insbesondere Polyhexamethylenbiguanid, sind antimikrobielle Wirkstoffe, die vor allem für die Konservierung kosmetischer Formulierungen, aber auch vermehrt in der Wundbehandlung, beispielsweise in Wundantiseptika, Wundspüllösungen und Wundauflagen eingesetzt werden.

Insbesondere im Bereich der Behandlung von Wunden, also auch bei Gegenständen, die mit Wunden in Berührung kommen, ist es von entscheidender Bedeutung, dass die zytotoxische Wirkung möglichst gering gehalten wird. Es haben sich dabei verschiedene Strategien im Stand der Technik etabliert, wie auf der einen Seite eine antimikrobielle Wirkung erzielt werden kann und gleichzeitig einer zytotoxischen Wirkung vorgebeugt werden kann.

EP-A1-1 175 148 offenbart polymere Wundauflagen aus Polyurethanschäumen, in deren Struktur der antimikrobielle Wirkstoff, beispielsweise das Polyhexamethylenbiguanid (PHMB), chemisch kovalent an die Polymerstruktur gebunden ist. Durch diese kovalente Bindung besteht nur eine eingeschränkte Bioverfügbarkeit und damit eine geringe Gefahr, zytotoxischer Effekte, allerdings bei gleichzeitig reduzierter antimikrobieller Wirksamkeit.

WO-A2-2007/089763 beschreibt superweiche Schaummaterialien für den Einsatz als Wundauflagen, die ein oder mehrere antimikrobiell wirksame Substanzen enthalten. Dabei werden diese Wirkstoffe in geeigneter Menge eingesetzt, so dass sie einen noch akzeptablen Wirkungsgrad zeigen, dabei aber nicht zytotoxisch wirken. Die Balance zwischen erwünschter antimikrobieller und unerwünschter zytotoxischer Wirksamkeit wird hier ausschließlich über die Auswahl einer geeigneten Konzentration des antimikrobiellen Wirkstoffs angestrebt.

EP-A1-1 830 869 beschreibt ein zelluläres, hydrophiles Polyurethan, enthaltend als antiseptischen Wirkstoff Polyhexamethylenbiguanid bzw. dessen Hydrochlorid, wobei das antiseptisch wirkende Agens mikropartikulär verteilt und/oder homogen gelöst vorliegt und das antiseptische Agens neben einem Superabsorber im Polyurethangel vorliegt. Die Wirkungsweise beruht hier vor allem darin, dass das keimhaltige Wundsekret in die Wundauflage aufgenommen und nicht wieder abgegeben wird, wodurch eine Wirkungssteigerung bei sehr niedrigen Konzentrationen des Antimikrobiums und einer offenbar sehr geringen Freisetzung erreicht wird.

WO-A1-2009/010068 offenbart eine Wundauflage, bestehend aus einem Polyurethanschaum, wobei der Schaum einen antimikrobiellen Wirkstoff enthält, der mit Hilfe von Wasser aus dem Schaum freigesetzt werden kann und gleichzeitig als Modulator der Flexibilität des Schaums dient. Ein Weg zur Vermeidung eventueller unerwünschter Nebeneffekte wird hier nicht aufgezeigt. DE-A1-10 2007 030 931 beschreibt Zusammensetzungen zur äußeren Behandlung von Wunden, die einen nutritiven, einen desinfizierenden und einen Protease hemmenden Stoff enthalten. Dabei wird allerdings kein Verfahren zur Senkung einer durch desinfizierende Inhaltsstoffe, wie Polihexanid möglichen zytótoxischen Wirkung beschrieben.

Insbesondere beim Einsatz solcher Wundauflagen, die den antimikrobiellen Wirkstoff, insbesondere Wirkstoffe auf Basis von Guanidinderivaten oder Biguanidderivaten freisetzen können, lag der vorliegenden Erfindung die Aufgabe zugrunde, eine Wundauflage zur Verfügung zu stellen, die zum Einen antimikrobiell wirksam ist und auf der anderen Seite gleichzeitig eine möglichst geringe zytotoxische Wirkung entfaltet.

Überraschend wurde gefunden, dass die im Stand der Technik aufgeführten Probleme gelöst werden können durch eine Wundauflage, die ein Substrat sowie eine Zusammensetzung mit mindestens einem antimikrobiellen Wirkstoff und mindestens einem die Zytotoxizität absenkenden Mittel enthält.

Gegenstand der vorliegenden Erfindung ist eine Wundauflage umfassend ein polymeres Substrat und eine Zusammensetzung umfassend
a) mindestens einen antimikrobiellen Wirkstoff und
b) ein die Zytotoxizität absenkendes Mittel, umfassend eine Öl in Wasser Emulsion, die zusätzlich ein oder mehrere Alkandiol(e) mit 3 bis 12 Kohlenstoffatomen aufweist.

Das polymere Substrat der erfindungsgemäßen Wundauflage ist bevorzugt derart ausgestaltet, dass es Flüssigkeit absorbieren kann. Das polymere Substrat liegt als zellulärer Schaum vor, beispielsweise mit einer schwammartigen Struktur.

Die Auswahl des polymeren Substrats unterliegt keinen Einschränkungen, solange es sich für den Kontakt mit Haut und Wunden eignet und hier insbesondere keine schädigende Wirkung hervorruft. Prinzipiell geeignet sind polymere Substrate natürlichen, wie auch künstlichen Ursprungs. Besonders bevorzugt wird das polymere Substrat ausgewählt aus der Gruppe bestehend aus Polyurethane, Polyether, Cellulosematerialien, Cellulosederivate, Baumwolle, Rayon, Polyester, Polyvinylalkohol, Polyvinylacetat, Polysulfone, Polyacrylate, Polyolefine, Polyamide, Alginate, Chitosan sowie beliebige Mischungen und Kombinationen hiervon.

Unter den Polyestern hat sich das Polyethylenterephthalat als besonders geeignet herausgestellt. Bei den Polyolefinen können sowohl Polyethylene, Polypropylene als auch beliebige Copolymere hiervon eingesetzt werden. Als geeignete Polyamide eignen sich beispielsweise Nylon, insbesondere Nylon 6,6 (das Polykondensationsprodukt von 1,6-Diaminohexan mit Adipinsäure) oder auch das Nylon 6, was auch als Polycaprolactam bezeichnet wird. Besonders bevorzugt im Rahmen der vorliegenden Erfindung ist das polymere Substrat ein Polyurethan, das erhältlich ist durch Umsetzung von einer Komponente, die mindestens eine Isocyanatgruppe, vorzugsweise zwei Isocyanatgruppen aufweist, mit mindestens einem Polyol, vorzugsweise einem Polyetherpolyol und/oder einem Polyesterpolyol.

Geeignete polymere Substrate, die für den Einsatz von Wundauflagen in Frage kommen, werden beispielsweise in der WO 2009/010068 A1 und der WO 2007/089763 A2 beschrieben.

Besonders bevorzugt ist ein vernetztes, hydrophiles Polyurethan, das saugfähig ist.

Besonders geeignet sind polymere Substrate in Form eines Polyurethanschaums. Die Polyurethanschäume können dazu durch NCO-terminierte Prepolymere in Kombination mit der erfindungsgemäß einzusetzenden, bevorzugt wässrigen Zusammensetzung, hergestellt werden. Das NCO-terminierte Prepolymer polymerisiert rasch in einer wässrigen Phase, die beispielsweise Tenside enthält, unter Ausbildung eines Schaums. Der Schaum wird bevorzugt bereits bei der Herstellung in Form einer Wundauflage gefertigt.

Im Rahmen der vorliegenden Erfindung sind für die Herstellung von Polyurethanschäumen besonders NCO-terminierte Polyetherprepolymere geeignet. Polyetherprepolymere umfassen dabei insbesondere hydrophile Polyetherpolyole. Geeignete hydrophile Polyetherpolyole umfassen dabei die Reaktionsprodukte von Ethylenoxid oder Kombinationen von Ethylenoxid mit anderen Alkylenoxiden mit einem oder mehreren Komponenten, die mindestens zwei aktive Wasserstoffatome aufweisen, wie beispielsweise Polyole, Polyphenole, Polyamine, Polycarbonsäuren, Phosphorsäuren und dergleichen. Beispiele geeigneter Polyole sind beispielsweise Ethylenglykol, Propylenglykol, 1,3- und 1,4-Butandiole, 1,6-Hexandiol, Diethylenglykol, Bis(hydroxymethyl)cyclohexan, Bis(hydroxyethyl)benzen, hydriertes Bisphenol A, hydriertes Bisphenol F, Polytetramethylenglykole, Polyesterdiole und Silanol terminierte Polysiloxane, Glycerin, Trimethylolpropan, Trimethylolethan, 1,2,3-Butantriol, 1,2,6-Hexantriol, Polyestertriole, Pentaerythrol, Diglycerol, α-Methylglucoside, Sorbitol, Xylitol, Mannitol, Glucose, Fructose, Succrose, und dergleichen. Beispiele geeigneter Phenole sind Hydrochinon, Catechol, Resorcin, Pyrrogalol und Bisphenole, wie beispielsweise Bisphenol A, Bisphenol F, Bisphenol S und dergleichen. Darüber hinaus kommen Ethanolamine sowie aliphatische, aromatische, alicyclische und araliphatische Polyamine, wie beispielsweise C₂-C₆-Alkylendiamine, Diethylentriamine, Tolouendiamine, Phenylendiamine, Xylylendiamine, Methylendiamine, Diphenyletherdiamine, Isophorondiamine, Cyclohexylendiamine, Cyclohexymethandiamine und dergleichen in Frage.

Geeignete Alkylenoxide, die in Kombination mit Ethlyenoxid zur Herstellung der Polyetherpolyole eingesetzt werden können, sind beispielsweise, Propylenoxid, 1,2-, 2,3-, 1,3- und 1,4-Butylenoxid, Styrenoxid, Epichlorhydrin und dergleichen. Die Zugabe des Ethylenoxids oder der Kombination des Ethylenoxids mit weiteren Alkylenoxiden zu den aktiven Wasserstoff tragenden Verbindungen, kann in dem Fachmann geläufigen Verfahren mit oder ohne Katalysatoren erfolgen. Die Zugabe der Ethylenoxide und Alkylenoxide kann dabei in zufälliger Form zur Ausbildung von Mischpolyethern oder in Blockform zur Ausbildung von Blockpolymeren erfolgen.

In einer bevorzugten Ausführungsform weisen die Polyole zur Herstellung der NCO-terminierten Prepolymere einen Oxyethylengehalt von mindestens 30 Gew.-%, weiter bevorzugt mindestens 50 Gew.-%, und insbesondere mindestens 90 Gew.-% auf und eine mittlere Hydroxygruppenzahl von 2 bis 8, insbesondere 2 bis 4 auf.

Die zuvor beschriebenen Polyetherpolyole werden anschließend mit Isocyanaten, beispielsweise aromatischen Isocyanaten oder aliphatischen Isocyanaten gekappt. Geeignete aromatische Isocyanate umfassen solche, die 6 bis 20 Kohlenstoffatome (nicht die Kohlenstoffatome der NCO-Gruppe beinhaltend) aufweisen. Geeignete Beispiele sind p-Phenylendiioscyanat (PDI), 4,4'-Diphenylmethandiisocyanat (MDI) und Positionsisomere hiervon, 2,4- und/oder 2,6-Toluoldiisocyanat (TDI) und Positionsisomere hiervon, 3,4-Dichlorphenyldiisocyanat, Dicyclohexylmethan-4,4'-diisocyanat (HMDI), 1,6-Hexamethylendiisocyanat (HDI) und Positionsisomere hiervon und dergleichen. Geeignete alphatische Isocyanate umfassen Isophorondiisocyanat (IPDI) und dergleichen.

Zur Herstellung der NCO-terminierten hydrophilen Urethanprepolymere werden die Isocyanate mit mindestens einem hydrophilen Polyetherpolyol, bevorzugt in einem Verhältnis zur Reaktion gebracht, so dass das Verhältnis von NCO/OH von 1,5 bis 5,0, weiter bevorzugt von 1,7 bis 3,0, vorliegt. Die Reaktion der Isocyanate mit den Polyetherpolyolen zur Ausbildung der Prepolymere, kann in dem Fachmann geläufigen Verfahren durchgeführt werden. In bevorzugten Ausführungsformen kann die Reaktion auch in Gegenwart von Katalysatoren durchgeführt werden.

Der NCO-Gehalt der NCO-terminierten hydrophilen Prepolymere beträgt bevorzugt 1 bis 10 Gew.-%, weiter bevorzugt 2 bis 8 Gew.-%. Geeignete NCO-terminierte Polyetherprepolymere, die im Rahmen der vorliegenden Erfindung für die Herstellung der Wundauflage verwendet werden können, sind beispielsweise in den Schriften US-3,903,232 und US-4,137,200 offenbart. Solche Prepolymere zeigen beispielsweise eine durchschnittliche Isocyanatfunktionalität von oberhalb 2, in manchen Ausführungsformen sogar eine Funktionalität von 2 bis 10. Geeignete "NCO-terminierte Polyetherprepolymere" umfassen auch solche, die unter dem Markennamen Hypol, wie beispielsweise Hypol 2000, Hypol 2002, Hypol 3000, Hypol 4000, Hypol 5000, Hypol X 6100 und Hypol Hydrogel kommerziell erhältlich sind. Bevorzugte NCO-terminierte Polyetherprepolymere zeigen ein Äquivalentgewicht (Molekulargewicht pro NCO-Gruppe) von 100 bis 1000 Dalton, bevorzugt 500 bis 750 Dalton.

In einer besonders bevorzugten Ausführungsform werden die Prepolymere für die Herstellung des Polyurethanschaums zusammen mit einer zuvor bereitgestellten wässrigen Zusammensetzung, die mindestens einen antimikrobiellen Wirkstoff und mindestens ein oder mehrere die Zytotoxizität absenkende Mittel aufweist vereinigt, unter Ausbildung des Polyurethanschaums.

In einer bevorzugten Ausführungsform ist das polymere Substrat ausgewählt aus der Gruppe bestehend aus Polyethylenterephthalat, Polyethylen, Polypropylen, Nylon-6,6, Polycaprolactam, Polyurethan sowie beliebigen Mischungen und Kombinationen hiervon.

In einer bevorzugten Ausführungsform ist das polymere Substrat ein Polyurethan, vorzugsweise ein bei einer Temperatur von 20 bis 45°C aufgeschäumtes Polyurethan oder ein thermoplastisches Polyurethan.

Ein weiterer wesentlicher Bestandteil der erfindungsgemäßen Wundauflage ist eine Zusammensetzung, die mindestens einen antimikrobiellen Wirkstoff und mindestens einen die Zytotoxizität absenkendes Mittel umfasst. Bevorzugt ist diese Zusammensetzung während des Polymerisationsverfahrens zur Herstellung des polymeren Substrates zugegen und wird dadurch in die Polymermatrix eingebunden. Alternativ kann die Zusammensetzung auf das polymere Substrat aufgebracht werden, beispielsweise durch Tauchen oder Besprühen. Sollte das polymere Substrat eine gelartige Konsistenz aufweisen, so lässt sich die Zusammensetzung nach dem Fachmann geläufigen Methoden in das Substrat einarbeiten. Bevorzugt ist die Zusammensetzung flüssig bei 20°C. Weiter bevorzugt handelt es sich bei der Zusammensetzung um eine wässrige Formulierung. Der pH-Wert der Zusammensetzung liegt bevorzugt bei pH 4 bis 8, insbesondere 5 bis 7.

Die Zusammensetzung umfasst als wesentlichen Bestandteil einen antimikrobiellen Wirkstoff.

Der antimikrobielle Wirkstoff ist bevorzugt ausgewählt aus der Gruppe der Komponenten, die mindestens eine Guanid- und/oder Biguanid Gruppe aufweisen, Iodophoren, Triclosan, Octenidin, quaternären Ammoniumverbindungen, Lactoferrin, Undecylensäure und/oder deren Salze und/oder beliebige Mischungen hiervon. Besonders bevorzugt ist der antimikrobielle Wirkstoff ausgewählt aus der Gruppe bestehend aus Polymethylenbiguanid, Polyhexamethylenbiguanid, Polyhexamethylenguanid, Chlorhexidin, Hexetidin, Iodophoren, PVP-Iod, 2,4,4'-Trichlor-2-hydroxydiphenylether, Octenidin, sowie deren Salze, vorzugsweise deren Hydrohalogenide, Acetate, Gluconate oder Sulfate, insbesondere Chlorhexidinacetat, Chlorhexidingluconat, Chlorhexidinsulfat, Chlorhexidinhydrochlorid, und Octenidinhydrochlorid.

Speziell bevorzugt ist der antimikrobielle Wirkstoff eine aliphatische Komponente, die eine oder mehrere Guanid- und/oder Biguanid-Gruppe(n) aufweist. Eine aliphatische Komponente im Rahmen der vorliegenden Erfindung bedeutet, dass die Komponente kein aromatisches Ringsystem aufweist. "Aliphatisch" im Rahmen der vorliegenden Erfindung umfasst auch cycloaliphatische oder auch heterocyclische Systeme, sofern diese gesättigte Ringsysteme aufweisen.

Im Rahmen der vorliegenden Erfindung handelt es sich um eine Komponente, die eine Guanid-Gruppe aufweist, wenn die Komponente in ihrer chemischen Struktur das folgende Strukturelement aufweist:

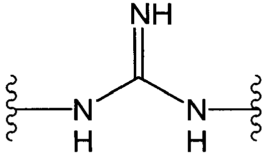

Nachfolgend werden diese Komponenten vereinfachend als "Guanid" oder "Guanide" bezeichnet.

Unter Komponenten, die eine Biguanidgruppe aufweisen sind solche chemische Verbindungen zu verstehen, die das folgende Strukturelement aufweisen:

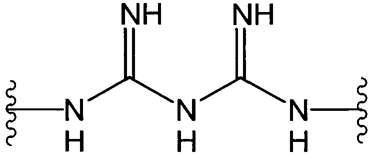

Nachfolgend werden diese Komponenten vereinfachend als "Biguanid" oder "Biguanide" bezeichnet.

Als besonders geeigneter antimikrobieller Wirkstoff hat sich das Polyhexamethylenbiguanid und/oder ein Salz des Polyhexamethylenbiguanids, vorzugsweise ein Hydrohalogenid, beispielsweise Hydrochlorid herausgestellt.

Überraschend hat sich auch gezeigt, dass antimikrobielle Wirkstoffe, die erhältlich sind durch Polykondensation eines Guanidinsäureadditionssalzes mit einem Amingemisch, welches wenigstens ein Diamin und/oder ein Triamin enthält, wobei vorzugsweise wenigstens ein Amin ausgewählt ist aus der Gruppe, die aus
i) Diamin, das wenigstens einen cycloaliphatischen Rest aufweist, und
ii) Dialkylentriamin besteht,
besonders geeignet sind.

Die Polykondensation kann dazu beispielsweise bei Temperaturen von 100°C bis 180°C, vorzugsweise 130°C bis 160°C über einen Zeitraum von bevorzugt 30 Minuten bis 6 Stunden durchgeführt werden.

Die so erhältlichen polymeren oder oligomeren antimikrobiellen Wirkstoffe können sowohl als Homopolymer als auch als Copolymer vorliegen. Vorteilhaft ist dabei, wenn das Guanidin-Säureadditionssalz Guanidiniumhydrochlorid ist. Es sind aber auch weitere Guanidin-Säureadditionssalze, auf Basis anorganischer oder organischer Säuren ebenfalls geeignet. Beispielsweise die Hydroxide, Hydrogensulfate sowie Acetate.

Diese antimikrobiellen Wirkstoffe zeichnen sich durch eine besonders hohe Biozidie aus und sind daher für die erfindungsgemäßen Wundauflagen besonders geeignet.

Bevorzugt sind die antimikrobiellen Wirkstoffe erhältlich durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
i) Diamin, das wenigstens einen cycloaliphatischen Rest aufweist, und
ii) Dialkylentriamin besteht.

Die durch die Polykondensation erhältlichen polymeren oder oligomeren Wirkstoffe weisen hierbei bevorzugt eine Polyguanidinstruktur oder, insbesondere im Falle des Einsatzes von Dialkylentriaminen, beispielsweise Diethylentriamin, eine Poly(iminoimidazol)-Struktur auf.

In einer bevorzugten Ausgestaltung der vorliegenden Erfindung umfasst das Amingemisch die Komponente i) (Diamin, das wenigstens einen cycloaliphatischen Rest aufweist) und/oder die Komponente ii) (Dialkylentriamin) in einer Menge von wenigstens 10 mol-%, bevorzugt mindestens 25 mol-%, weiter bevorzugt mindestens 45 mol-%, insbesondere mindestens 85 mol-%, im Speziellen mindestens 95 mol-%, jeweils bezogen auf das gesamte Amingemisch.

Bevorzugt umfasst das Amingemisch zusätzlich ein Alkylendiamin, das besonders bevorzugt eine Verbindung der allgemeinen Formel

NH₂(CH₂)ₙNH₂

in welcher n eine ganze Zahl zwischen 2 und 10, bevorzugt 4 oder 6, ist. Bevorzugt einzusetzende Alkylendiamine tragen endständige Aminogruppen. Speziell bevorzugt ist das Hexamethylendiamin (Hexan-1,6-diamin). Das Alkylendiamin kann in der Polykondensationsreaktion in Mischung mit weiteren Diaminen oder Triaminen, wobei wenigstens ein Amin ausgewählt ist aus der Gruppe, die aus
i) Diamin, das wenigstens einen cycloaliphatischen Rest aufweist, und
ii) Dialkylentriamin besteht,
bevorzugt ausgewählt aus der Gruppe bestehend aus 4,4'-Methylenbis(cyclohexylamin) und/oder Diethylentriamin unter Ausbildung von Copolymeren eingesetzt werden.

Vorzugsweise kann das Amingemisch weiterhin Oxyalkylendiamine umfassen.

Als Oxyalkylendiamine eignen sich insbesondere solche Oxyalkylendiamine, die endständige Aminogruppen aufweisen. Ein bevorzugtes Oxyalkylendiamin ist eine Verbindung der allgemeinen Formel

NH₂[(CH₂)₂O)]ₙ(CH₂)₂NH₂

in welcher n eine ganze Zahl zwischen 2 und 6, bevorzugt zwischen 2 und 5, weiter bevorzugt zwischen 2 und 4 und insbesondere 2 ist. Bevorzugt sind Polyoxyethylendiamine, im Speziellen Triethylenglycoldiamin. Weiter bevorzugt eingesetzt werden können Polyoxypropylendiamine, insbesondere Di- oder Tripropylenglycoldiamin.

In einer bevorzugten Ausführungsform liegt der polymere oder oligomere Wirkstoff als Homopolymer vor. In diesen Fällen besteht das Amingemisch aus einem Diamin, das wenigstens einen cycloaliphatischen Rest aufweist, oder aus einem Dialkylentriamin.

In einer weiteren bevorzugten Ausführungsform besteht das Amingemisch aus dem Triamin Diethylentriamin. In dieser Variante liegt der polymere oder oligomere Wirkstoff somit als Homopolymer vor, beispielsweise als Poly(iminoimidazolidin).

In einer weiteren bevorzugten Ausführungsform besteht das Amingemisch aus dem Diamin 4,4'-Methylenbis(cyclohexylamin). Durch Polykondensation mit einem Guanidinsäureadditionssalz entsteht daraus beispielsweise das Homopolymer Poly(4,4'-methylenbis(cyclohexylaminhydrochlorid)).

Besonders bevorzugt sind die polymeren oder oligomeren Wirkstoffe, die erhältlich sind durch Polykondensation eines Guanidinsäureadditionssalzes mit einem Amingemisch, welches wenigstens ein Diamin, das wenigstens einen cycloaliphatischen Rest aufweist, umfasst. Diamine, die wenigstens einen cycloaliphatischen Rest aufweisen, sind beispielsweise cycloaliphatische Diamine, beispielsweise Cyclohexandiamin, Cyclopentandiamin sowie Derivate hiervon. Insbesondere bevorzugt sind solche Diamine, bei denen wenigstens eine NH₂-Gruppe direkt mit dem cycloaliphatischen Rest verknüpft ist. Besonders bevorzugt sind solche Diamine, bei denen beide NH₂-Gruppen jeweils direkt mit ein und demselben cycloaliphatischen Rest oder an verschiedenen cycloaliphatischen Resten verknüpft sind. In einer besonderen Ausführungsform umfasst das Amingemisch 4,4'-Methylenbis(cyclohexylamin).

In einer weiteren Ausführungsform der vorliegenden Erfindung umfasst das Amingemisch wenigstens ein Dialkylentriamin. Die Dialkylentriamine können dabei Alkylenreste unterschiedlicher Kettenlänge aufweisen. Bevorzugt sind allerdings Dialkylentriamine, bei denen die Alkylengruppen die gleiche Länge aufweisen. Bevorzugte Alkylenreste sind dabei Ethylen, Propylen und Butylen sowie Hexylen. In einer besonders bevorzugten Ausführungsform umfasst das Amingemisch das Triamin Diethylentriamin.

In einer weiteren bevorzugten Ausführungsform liegen die erfindungsgemäß verwendeten polymeren oder oligomeren Wirkstoffe in Form von Copolymeren vor. Diese können dabei sowohl willkürlich gemischt als auch als Blockcopolymere vorliegen. Im Falle von Copolymeren enthält das Amingemisch wenigstens zwei verschiedene Amine. Das Amingemisch enthält dabei eine erste Komponente und wenigstens eine zweite Komponente, wobei bevorzugt
die erste Komponente ein Diamin oder ein Triamin ist, das ausgewählt ist aus der Gruppe, die aus
i) Diamin, das wenigstens einen cycloaliphatischen Rest aufweist, und
ii) Dialkylentriamin besteht, und wobei
die zweite Komponente ein Diamin oder ein Triamin ist, das ausgewählt ist aus der Gruppe, die aus
i) Diamin, das wenigstens einen cycloaliphatischen Rest aufweist,
ii) Dialkylentriamin,
iii) Alkylendiamin und
iv) Oxyalkylendiamin besteht, und
wobei die erste Komponente von der zweiten Komponente verschieden ist.

Als besonders geeignete copolymere oder cooligomere Wirkstoffe haben sich solche herausgestellt, bei denen die erste Komponente 4,4'-Methylenbis(cyclohexylamin) ist und die zweite Komponente ausgewählt ist aus Diethylentriamin, Hexamethylendiamin und Triethylenglycoldiamin.

In einer weiteren bevorzugten Ausführungsform enthält das copolymere Guanidinderivat als erste Komponente Diethylentriamin und die zweite Komponente ist ausgewählt aus der Gruppe bestehend aus Hexamethylendiamin und Triethylenglycoldiamin.

Die erfindungsgemäße Wundauflage enthält besonders bevorzugt eine Zusammensetzung, die einen antimikrobiellen Wirkstoff mit einem mittleren Molekulargewicht im Bereich von 500 bis 7000, insbesondere 1000 bis 5000 Dalton aufweist.

Im Rahmen der vorliegenden Erfindung wird der Begriff polymeres Guanidinderivat oder Bigunaid für Guanidinderivate bzw. Biguanide verwendet in denen 2 oder mehr Repetitionseinheiten auftreten. Der Begriff "Polymer" erfasst somit auch bereits Dimere, Trimere oder beispielsweise Oligomere.

Eine weitere Klasse polymerer Guanidinderivate ist beispielsweise in der WO-A1-01/85676 sowie in der WO-A1-06/047800 beschrieben.
Bevorzugte antimikrobielle Wirkstoffe sind dabei erhältlich durch Polykondensation eines Guanidin-Säureadditionssalzes mit einem Amingemisch, das wenigstens ein Diamin enthält, welches ausgewählt ist aus der Gruppe bestehend aus Aklylendiamin und Oxyalkylendiamin.
Bevorzugt umfasst hierbei das Amingemisch ein Alkylendiamin, das besonders bevorzugt eine Verbindung der allgemeinen Formel

NH₂(CH₂)ₙNH₂

in welcher n eine ganze Zahl zwischen 2 und 10, bevorzugt 4 oder 6, ist. Bevorzugt einzusetzende Alkylendiamine tragen endständige Aminogruppen. Speziell bevorzugt ist das Hexamethylendiamin (Hexan-1,6-diamin). Das Alkylendiamin kann in der Polykondensationsreaktion in Mischung mit weiteren Polyaminen, beispielsweise Di- und/oder Triaminen unter Ausbildung von Copolymeren eingesetzt werden.

Vorzugsweise umfasst das Amingemisch wenigstens ein Oxyalkylendiamin.

Als Oxyalkylendiamine eignen sich insbesondere solche Oxyalkylendiamine, die endständige Aminogruppen aufweisen. Ein bevorzugtes Oxyalkylendiamin ist eine Verbindung der allgemeinen Formel

NH₂[(CH₂)₂O)]ₙ(CH₂)₂NH₂

in welcher n eine ganze Zahl zwischen 2 und 6, bevorzugt zwischen 2 und 5, weiter bevorzugt zwischen 2 und 4 und insbesondere 2 ist. Bevorzugt sind Oxyethylendiamine, im Speziellen Diethylenglycoldiamin oder Triethylenglycoldiamin. Weiter bevorzugt eingesetzt werden können Polyoxypropylendiamine, insbesondere Di- oder Tripropylenglycoldiamin.

Bevorzugt liegt das polymere Guanidinderivat als Homopolmyer vor. In diesen Fällen besteht das Amingemisch aus dem Alkylendiamin oder aus einem Oxyalkylendiamin.

In einer weiteren bevorzugten Ausführungsform besteht das Amingemisch aus dem Alkylendiamin Hexamethylendiamin (Hexan-1,6-diamin). In dieser Variante besteht das polymere Guanidinderivat somit aus einem Homopolymer, beispielsweise das Poly-(hexamethylen-guanidinium-chlorid) (PHMG).

In einer weiteren bevorzugten Ausführungsform besteht das Amingemisch aus dem Oxyalkylendiamin Triethylenglycoldiamin. Durch Polykondensation mit einem Guanidinsäureadditionssalz entsteht daraus beispielsweise das Homopolymer Poly-[2-(2-ethoxy)-ethoxyethyl)-guanidinium-chlorid].

In einer weiteren bevorzugten Ausführungsform liegen die erfindungsgemäß verwendeten polymeren Guanidinderivate in Form von Copolymeren vor. Diese können dabei sowohl willkürlich gemischt als auch als Blockcopolymere vorliegen. Im Falle von Copolymeren enthält das Amingemisch wenigstens zwei verschiedene Amine. Das Amingemisch enthält dabei eine erste Komponente und wenigstens eine zweite Komponente, wobei
- die erste Komponente ein Diamin ist, ausgewählt aus der Gruppe bestehend aus Alkylendiamin und Oxyalkylendiamin, und wobei
- die zweite Komponente ein Diamin ist, das ausgewählt aus der Gruppe bestehend aus Alkylendiamin und Oxyalkylendiamin, und
wobei die erste Komponente von der zweiten Komponente verschieden ist.

Als besonders geeignete copolymere Guanidinderivate haben sich solche herausgestellt, bei denen die erste Komponente Alkylendiamin und die zweite Komponente ein Oxyalkylendiamin ist. Besonders bevorzugt sind copolymere Guanidinderivate bei denen im Amingemisch die erste Komponente Hexamethylendiamin und die zweite Komponente Triethylenglycoldiamin ist.

Bei der Herstellung von Copolymeren kann das Mischungsverhältnis der einzusetzenden Amine in weiten Bereichen variiert werden. Bevorzugt sind allerdings copolymere Guanidinderivate, bei denen die Monomere des Amingemischs, also die erste Komponente und die zweite Komponente, in einem Molverhältnis von 4 : 1 bis 1 : 4, vorzugsweise 2 : 1 bis 1 : 2, vorliegt.

Die erfindungsgemäß einzusetzenden polymeren Guanidinderivate besitzen bevorzugt ein mittleres Molekulargewicht (Gewichtsmittel) im Bereich von 500 bis 7000, insbesondere von 1000 bis 5000 Dalton.

In einer weiteren bevorzugten Ausführungsform der vorliegenden Erfindung liegt das erfindungsgemäß einzusetzende polymere Guanidinderivat als Mischung aus mindestens 2 unterschiedlichen polymeren Guanidinderivaten vor. In einer speziellen Ausführungsform umfasst die Mischung der polymeren Guanidinderivate sowohl ein erstes Homopolymer basierend auf einem Alkylendiamin, vorzugsweise Poly-(hexamethylen-guanidinium-chlorid) als auch ein zweites Homopolymer basierend auf einem Oxyalkylendiamin, beispielsweise Poly-[2-(2-ethoxy)-ethoxyethyl)-guanidinium-chlorid].

In einer bevorzugten Ausführungsform umfasst das polymere Guanidinderivat das erste Homopolymer und das zweite Homopolymer in einem Gewichtsverhältnis von 5:1 bis 1:5, vorzugsweise 1:1 bis 1:4 und insbesondere 1:2 bis 1:4. In einer besonders bevorzugten Ausführungsform umfasst das polymere Guanidingemisch Poly-(hexamethylen-guanidinium-chlorid) (erstes Homopolymer) und Poly-[2-(2-ethoxy)-ethoxyethyl)-guanidinium-chlorid] (zweites Homopolymer) in einem Gewichtsverhältnis (erstes Homopolymer zu zweitem Homopolymer) von 1:1 bis 1:5, vorzugsweise 1:2 bis 1:4, insbesondere 1:3. Solche Mischungen eignen sich insbesondere für die Wundauflage der vorliegenden Erfindung.

Die erfindungsgemäß verwendeten polymeren Guanidinderivate und Biguanide können allgemein sowohl als Homopolymer als auch als Copolymer vorliegen. Vorteilhaft ist dabei, wenn das Guanidin-Säureadditionssalz Guanidiniumhydrochlorid ist. Es sind aber auch weitere Guanidin-Säureadditionssalze, auf Basis anorganischer oder organischer Säuren ebenfalls geeignet. Beispielsweise die Hydroxide, Hydrogensulfate sowie Acetate. Besonders geeignete und wirksame polymere Guanidinderivate liegen in Form ihrer Hydroxid-Salze vor. Diese können beispielsweise durch Anionenaustausch aus den korrespondierenden Chlorid-Salzen erhalten werden.

Die Zusammensetzung enthält außerdem ein die Zytotoxizität absenkendes Mittel, umfassend eine Öl in Wasser Emulsion, die zusätzlich ein oder mehrere Alkandiol(e) mit 3 bis 12 Kohlenstoffatomen aufweist.
Bevorzugt enthält die Zusammensetzung darüber hinaus weitere die Zytotoxizität absenkende Mittel die ausgewählt sind aus der Gruppe bestehend aus
i) einem oder mehreren Betainderivat(en),
ii) einem oder mehreren Fettalkoholalkoxylat(en),
iii) einem oder mehreren Glycerinester(n),
iv) Allantoin, und
v) Panthenol und/oder Pantothensäure.

Die Komponenten i) bis v) können in der Zusammensetzung jeweils einzeln oder in beliebigen Kombinationen vorliegen.

Ein wesentlicher Bestandteil der Zusammensetzung umfasst eine Öl in Wasser Emulsion.

Es hat sich überraschend gezeigt, dass Öl in Wasser Emulsionen die Zytotoxizität der eingesetzten antimikrobiellen Wirkstoffe erheblich absenken bzw. vollkommen ausschalten können. In einer bevorzugten Ausführungsform umfasst die Öl in Wasser Emulsion mindestens ein Phospholipid und mindestens eine Ölkomponente.

Prinzipiell eignen sich für die Öl in Wasser Emulsionen jegliche Ölkomponenten, bevorzugt sind jedoch Ölkomponenten natürlichen Ursprungs. Besonders bevorzugte Ölkomponenten sind Triglyceride, die Fettsäurereste tragen. Es hat sich als besonders vorteilhaft herausgestellt, insbesondere bezüglich der physiologischen Verträglichkeit, wenn die Ölkomponente ein mittelkettiges Triglycerid (MCT) umfasst. Bevorzugt weist das mittelkettige Triglycerid eine Struktur auf, die ein Glycerin ist, welches mit Fettsäuren verestert ist, die 6 bis 14 Kohlenstoffatome aufweisen. Besonders bevorzugt ist das mittelkettige Triglycerid, ein Glycerin, welches mit Fettsäuren verestert ist, die zumindest 90 mol.-% aus Caprylsäure (C₈) und Caprinsäure (C₁₀) bestehen.

Weiterhin bevorzugt sind als Ölkomponente Pflanzenöle einzusetzen. Besonders geeignet für die Öl in Wasser Emulsionen der vorliegenden Erfindung sind die Pflanzenöle ausgewählt aus der Gruppe bestehend aus Sojabohnenöl und Saffloröl oder Mischungen hiervon. Ein weiteres geeignetes Öl ist Triisostearin.

Es hat sich auch gezeigt, dass auch Mischungen von verschiedenen Ölen Vorteile zeigen. So ist etwa die Mischung aus mittelkettigem Triglycerid und zusätzlichem Pflanzenöl, insbesondere Sojabohnenöl und/oder Saffloröl, besonders physiologisch verträglich und zeigt ein optimales Wirkungsspektrum. In einer bevorzugten Ausführungsform liegt das Gewichtsverhältnis von mittelkettigem Triglycerid zu Pflanzenöl bei 1:10 bis 10:1, vorzugsweise 5:1 bis 1:5, weiter bevorzugt 2:1 bis 1:2, besonders bevorzugt 1,5:1 bis 1:1,5 und im Speziellen bei 1:1.

Die Öl in Wasser Emulsion umfasst die Ölkomponente üblicherweise in einer Menge von 1 bis 30 Gew.-%, vorzugsweise 2 bis 20 Gew.-%, insbesondere 4 bis 15 Gew.-% und im Speziellen von 5 bis 10 Gew.-%, jeweils bezogen auf das Gesamtgewicht der Emulsion.

Als weitere Komponenten können die Öl in Wasser Emulsionen mindestens ein Phospholipid umfassen.

Phospholipide sind phosphorhaltige, amphiphile Lipide. Sie sind im Organismus als Membranlipide am Aufbau der Doppellipidschicht einer Biomembran beteiligt. Sie setzen sich aus einem hydrophilen Kopf und zwei hydrophoben Kohlenwasserstoffschwänzen zusammen.

Bevorzugt enthält die Öl in Wasser Emulsion ein Phospholipid, das üblicherweise ausgewählt ist aus der Gruppe bestehend aus Phosphatidylcholin, Phosphatidylethanolamin, Phosphatidylserin, Phosphatidylinositol und Sphingomyelin sowie beliebige Mischungen hiervon.

Besonders bevorzugt enthält die Emulsion ein Lecithin, das insbesondere von Sojabohnen und/oder Eiern stammt.

Lecithin ist der klassische Name für eine Gruppe chemischer Verbindungen, die sogenannten Phosphatidylcholine. Dabei handelt es sich um Lipide, genauer Phospholipide, die aus Fettsäuren, Glycerin, Phosphorsäure und Cholin zusammengesetzt sind. Lecithine sind Bestandteile der Zellmembranen tierischer und pflanzlicher Lebewesen.

In einer besonders bevorzugten Ausführungsform weist die Emulsion Lecithin auf, welches mit natürlichen Fettsäuren verestert ist. Zur Erhöhung der Stabilität gegen oxidativen Verderb können die Fettsäuren des Lecithins hydriert sein. So lässt sich z.B. hydriertes Sojalecithin verwenden, welches im Handel unter der Bezeichnung Emulmetik 320 (Lucas Meyer Cosmetic S.A.) erhältlich ist

Bevorzugt enthalten die Öl in Wasser Emulsionen die Phospholipide üblicherweise in einer Menge von 0,1 bis 5 Gew.-%, vorzugsweise 0,2 bis 2,5 Gew.-%, weiter bevorzugt 0,3 bis 1,5 Gew.-% und insbesondere 0,4 bis 1,2 Gew.-%, jeweils bezogen auf das Gesamtgewicht der Emulsion.

Insbesondere bezüglich der verminderten Zytotoxizität hat es sich als vorteilhaft herausgestellt, das Gewichtsverhältnis des antimikrobiellen Wirkstoffs zu Phospholipid aufeinander abzustimmen. In einer bevorzugten Ausführungsform ist das Gewichtsverhältnis des antimikrobiellen Wirkstoffs zu Phospholipid 1:4 bis 1:40, vorzugsweise 1:6 bis 1:20 und insbesondere 1:8 bis 1:12.

Weiterhin ist es vorteilhaft, insbesondere im Hinblick auf die Ausbildung geeigneter Öl in Wasser Emulsionen, die die Bedingungen für geringe Zytotoxizität erfüllen, dass das Gewichtsverhältnis des antimikrobiellen Wirkstoffs zur Ölkomponente bei 1:80 bis 1:1000, vorzugsweise 1:100 bis 1:800, weiter bevorzugt 1:150 bis 1:600 und insbesondere 1:180 bis 1:450 liegt.

Es hat sich als besonders vorteilhaft herausgestellt, wenn die erfindungsgemäßen Wundauflagen eine Zusammensetzung aufweisen, welche die zuvor beschriebene Öl in Wasser Emulsion und als antimikrobiellen Wirkstoff Komponenten enthalten, die mindestens eine Guanid- und/oder Biguanid-Gruppe aufweist.

Der antimikrobielle Wirkstoff liegt üblicherweise in einer Menge von bis zu maximal 10 Gew.-%, insbesondere 0,25 bis 5 Gew.-% und im Speziellen in einer Menge von 0,3 bis 4 Gew.-%, bezogen auf die die Zytotoxizität vermindernden Zusammensetzung, vor.

Es wurde weiter überraschend gefunden, dass die Partikelgröße der emulgierten Öltröpfchen vorteilhafterweise im nanoskaligen Bereich einzustellen ist. In einer bevorzugten Ausführungsform der vorliegenden Erfindung weisen die emulgierten Öltröpfchen eine mittlere Partikelgröße von bis zu 300 nm, vorzugsweise 30 bis 260 nm, weiter bevorzugt 50 bis 100 nm auf. Die Bestimmung der Teilchengröße erfolgt mit Photonenkorrelationsspektroskopie (Autosizer II Malvern Instruments UK) bei 20°C.

Die Öl in Wasser Emulsion enthält zusätzlich ein oder mehrere Alkandiol(e)mit 3 bis 12 Kohlenstoffatomen. Der Einsatz von Alkandiolen mit 3 bis 12 Kohlenstoffatomen in den die Zytotoxizität absenkenden Zusammensetzung ist besonders bevorzugt.

Dabei kann der Einsatz von Alkandiolen mit 3 bis 12 Kohlenstoffatomen die Wundheilung fördern und sogar zu einer Verstärkung der Wirksamkeit gegen bestimmte Keime, wie beispielsweise Hefen (Candida) beitragen. Darüber hinaus wurde festgestellt, dass durch Einsatz von Alkandiolen mit 3 bis 12 Kohlenstoffatomen in den Öl in Wasser Emulsionen die Konzentration der antimikrobiellen Wirkstoffe abgesenkt werden kann bei gleichbleibender antimikrobieller Wirkung im Vergleich zu Emulsionen ohne Alkandiolen. Dies führt zusätzlich zu einer weiteren Herabsetzung der Zytotoxizität.

Als geeignete Alkandiole haben sich solche herausgestellt, die 3 bis 12 Kohlenstoffatome aufweisen. Die Alkandiole sind ausgewählt aus der Gruppe bestehend aus 1,2 Propylenglykol, 1,3-Butylenglykol, 1,2-Pentandiol, 1,2-Hexandiol, 1,6-Hexandiol und/oder 1,2-Octandiol. Bevorzugt sind 1,2-Alkandiole mit 5 bis 10 Kohlenstoffatomen. Insbesondere bevorzugt ist das 1,2 Octandiol.

Die Alkandiole werden bevorzugt in Mengen von 0,01 bis 10 Gew.-%, insbesondere 0,05 bis 5 Gew.-%, jeweils bezogen auf das Gesamtgewicht des polymeren Substrats und der Zusammensetzung, eingesetzt.

Glycerinether können ebenfalls bevorzugt in den Öl in Wasser Emulsionen enthalten sein. Sie zeigen einen, insbesondere auch im Zusammenwirken mit der Öl in Wasser Emulsion Zytotoxizität absenkenden Effekt.

Die nachfolgend aufgeführten Komponenten entsprechen der INCI Nomenklatur. Bevorzugt sind die Glycerinether ausgewählt aus der Gruppe bestehend aus Octoxyglycerin, Polyglyceryl-3 Methylglucose Distearat, Polyglyceryl-6 Polyhydroxystearat, Polyglyceryllaurat und Polyglycerylcaprat.

Bevorzugte Glycerinether sind auch die Glycerinmonoalkylether, wie beispielsweise 1-(2-Ethylhexyl)glycerinether.

Die Glycerinether werden bevorzugt in Mengen von 0,01 bis 10 Gew.-%, insbesondere 0,1 bis 5 Gew.-%, jeweils bezogen auf das Gesamtgewicht des polymeren Substrats und der Zusammensetzung, eingesetzt.

### i) Betainderivate

Ergänzend kann die Zusammensetzung als das die Zytotoxizität absenkende Mittel ein oder mehrere Betainderivat(e) mit oberflächenaktiver Wirkung enthalten. Neben der günstigen, die Zytotoxizität absenkende Wirkung der antimikrobiellen Substanzen, tragen die Betainderivate zudem in vorteilhafter Weise zur Aufweichung und Auflösung von Wundbelägen, zur Entfernung von Biofilmen sowie zur Feuchthaltung der Wunde bei. Darüber hinaus leisten die Betainderivate zusätzlich einen entzündungshemmenden Beitrag. Insbesondere vorteilhaft haben sich aus der Gruppe der Betainderivate die Alkylbetaine und Alkylamidopropylbetaine, insbesondere Undecylenamidopropylbetain (chemischer Name: (Carboxymethyl)dimethyl[3-[(1-Oxoundecenyl)amino]-propyl]ammoniumhydroxid) aber auch Cocosamidopropylbetain, Capryl/Capramidopropyl Betain, Ricinolamidopropylbetain, Laurylbetain sowie das Lauryldimethylbetain herausgestellt.

Die Betainderivate werden bevorzugt in Mengen von 0,01 bis 10 Gew.-%, insbesondere 0,05 bis 5 Gew.-%, jeweils bezogen auf das Gesamtgewicht des polymeren Substrats und der Zusammensetzung, eingesetzt.

### ii) Fettalkoholalkoxylate

Die nachfolgenden Fettalkoholalkoxylate werden entsprechend der INCI Nomenklatur, falls abweichend von der IUPAC-Nomenklatur bezeichnet.

Ergänzend können als die Zytotoxizität absenkendes Mittel Fettalkoholalkoxylate in der Zusammensetzung vorliegen. Bevorzugt handelt es sich bei den Fettalkoholalkoxylaten um C₁₂-C₁₈-Fettalkohole, die mit durchschnittlich 2 bis 15 Ethylenoxid- und/oder Propylenoxid-Einheiten verethert sind. Geeignete Beispiele sind hier Laureth-3 (C₁₂ Fettalkoholethoxylat 3 EO), Laureth-7 (C₁₂ Fettalkoholethoxylat 7 EO), Laureth-9 (C₁₂ Fettalkoholethoxylat 9 EO), Ceteareth-25 (C_{16/18} Fettalkoholethoxylat 25 EO), C_{12/14} Fettalkoholalkoxylat 2 EO+4 PO, C_{12/14} Fettalkoholalkoxylat 3 EO+6 PO, C_{12/14} Fettalkoholalkoxylat 5 EO+4 PO, Ceteareth-60 Myristyl Glycol C₁₆₋₁₈-Alkylpolyethyleneglycol tetradecylenglycolether), PPG-3 Stearylether (Stearylalkohol 3 PO) und PPG-15 Stearyl Ether (Stearylalkohol 15 PO).

Die "EO" und "PO"-Gruppe steht für die Anzahl der Ethylenglycolethergruppen (EP) bzw. die Anzahl der Propylenglycolethergruppen (PO).

Die Fettalkoholalkoxylate, werden bevorzugt in Mengen von 0,01 bis 10 Gew.-%, insbesondere 0,05 bis 5 Gew.-%, jeweils bezogen auf das Gesamtgewicht des polymeren Substrats und der Zusammensetzung, eingesetzt.

### iii) Glycerinester

Die nachfolgend aufgeführten Komponenten entsprechen der INCI Nomenklatur.

Ergänzend können als die Zytotoxizität absenkendes Mittel Glycerinester in der Zusammensetzung vorliegen.

Bevorzugt sind die Glycerinester ausgewählt aus der Gruppe bestehend aus Glycerinfettsäureestern, insbesondere Glycerylcaprat und Glyceryllaurat.

Die Glycerinester werden bevorzugt in Mengen von 0,01 bis 10 Gew.-%, insbesondere 0,1 bis 5 Gew.-%, jeweils bezogen auf das Gesamtgewicht des polymeren Substrats und der Zusammensetzung, eingesetzt.

### iv) Allantoin

Zusätzlich kann als die Zytotoxizität absenkendes Mittel Allantoin in die Zusammensetzung eingesetzt werden. Es hat sich überraschend gezeigt, dass Allantoin nicht nur die Zytotoxizität der antimikrobiellen Wirkstoffe herabsetzt, sondern zusätzlich den Zellaufbau und die Zellregeneration beschleunigt und somit die Wundheilung unterstützt. Das Allantoin (chemischer Name: N-(2,5-Dioxo-4-imidazolidinyl)-harnstoff) ist bei verschiedenen Tierarten, vor allem bei Säugetieren, neben der Harnsäure das Endprodukt des Abbaus von Nucleinsäuren, speziell von Purinbasen. Allantoin ist zudem in manchen heimischen Pflanzen, besonders im Beinwell, enthaltender Wirkstoff. Zudem lässt sich Allantoin in Schwarzwurzel, aber auch in Weizenkeimlingen, Sojakeimlingen, Reis, Blumenkohl, grünen Bohnen und der Rosskastanie finden. Das Allantoin wird bevorzugt in Mengen von 0,05 bis 2 Gew.-%, insbesondere 0,1 bis 1 Gew.-%, jeweils bezogen auf das Gesamtgewicht des polymeren Substrats und der Zusammensetzung, eingesetzt.

### v) Panthenol oder Pantothensäure

Ergänzend kann als die Zytotoxizität absenkendes Mittel Panthenol und/oder Pantothensäure in die Zusammensetzung eingesetzt werden. Das Panthenol unterstützt dabei zusätzlich die Neubildung von Hautzellen und wirkt zudem entzündungshemmend. Das Panthenol wird auch als Dexpanthenol bezeichnet, welches sich im Körper zu Pantothensäure umwandelt. Pantothensäure ist ein Vitamin aus der Gruppe der B-Vitamine (Vitamin B5).

Das Panthenol und/oder die Pantothensäure liegen bevorzugt in einer Menge von 0,05 bis 5 Gew.-%, weiter bevorzugt 0,1 bis 2 Gew.-% und insbesondere von 0,2 bis 1 Gew.-%, jeweils bezogen auf das Gesamtgewicht des polymeren Substrats und der Zusammensetzung, vor.

Darüber hinaus kann die Zusammensetzung weitere Komponenten enthalten, die die Wundheilung und/oder den Zellaufbau begünstigen bzw. die Zytotoxizität verringern können. Hier haben sich Polyalkylenglykole als besonders geeignet erwiesen. Ergänzend kann die Zusammensetzung daher Polyalkylenglykole aufweisen.

Polyalkylenglykole (Polyglykole, Polyglykolether; INCI Chemical Class: Polymeric Ethers) sind bekannte, überwiegend lineare, z. T. aber auch verzweigte Polyether, bei denen es sich um Polymere mit endständigen Hydroxy-Gruppen handelt. Die Polyalkylenglykole mit höheren Molmassen sind polymolekular, d. h. sie bestehen aus Kollektiven von Makromolekülen mit unterschiedlichen Molmassen.

Die mittleren relativen Molmassen der Polyalkylenglykole liegen vorzugsweise im Bereich von 200 bis 10000, insbesondere von 500 bis 8000, besonders bevorzugt von 1000 bis 6000 und äußerst bevorzugt von 1500 bis 5000. Bei den nachfolgend beschriebenen verschiedenen Polyalkylenglykolen können bestimmte Bereiche nochmals besonders vorteilhaft sein.

Erfindungsgemäß bevorzugt sind lineare oder verzweigte, insbesondere lineare Polyalkylenglykole der allgemeinen Formel HO-[R-O]ₙ-H, in der R für (CH₂)₂, CH₂CH(CH₃), CH₂CH(CH₂CH₃), und/oder für (CH₂)₄ und n für Werte bzw. Mittelwerte von 2 bis etwa 200, vorzugsweise 3 bis 190, weiter bevorzugt 4 bis 180, besonders bevorzugt 6 bis 150, insbesondere 10 - 120 stehen. Die Polyalkylenglykole können durch ringöffnende Polymerisation von Ethylenoxid, Propylenoxid und/oder Tetrahydrofuran hergestellt werden. Es sind dies im Einzelnen die Polyethylenglykole mit R = (CH₂)₂, die Polypropylenglykole mit R = CH₂CH(CH₃), die Polytetrahydrofurane mit R = (CH₂)₄ und die Copolymere aus Ethylenoxid, Propylenoxid und/oder Tetrahydrofuran.

Erfindungsgemäß bevorzugt sind Polyethylenglykole (PEG) mit einer mittleren relativen Molmasse von 400 bis 10000, insbesondere von 1000 bis 8000, besonders bevorzugt von 2000 bis 6000 und äußerst bevorzugt von 3000 bis 5000. Für Polyethylenglykole existieren verschiedene Nomenklaturen, die zu Verwirrungen führen können. Technisch gebräuchlich ist die Angabe des mittleren relativen Molgewichts im Anschluss an die Angabe "PEG", so dass "PEG 200" ein Polyethylenglykol mit einer relativen Molmasse von ca. 190 bis ca. 210 charakterisiert. Gemäß INCI-Nomenklatur wird das Kurzzeichen PEG mit einem Bindestrich versehen und folgt direkt auf den Bindestrich eine Zahl, die der Zahl n in der obigen allgemeinen Formel entspricht. Kommerziell erhältliche Polyethylenglykole sind beispielsweise PEG 200/PEG-4, PEG 300/PEG-6, PEG- 7, PEG-8, PEG 400, PEG-9, PEG-10, PEG-12, PEG 600, PEG-14, PEG-16, PEG 800/PEG-18, PEG-20, PEG 1000, PEG 1200, PEG 1500/PEG-32, PEG-40, PEG 2000, PEG-55, PEG-60, PEG 3000, PEG 3350/PEG-75 und PEG 4000/PEG-90, wobei die Bezeichnungen gemäß den beiden Nomenklaturen für einander entsprechende Polyethylenglykole durch das Zeichen "/" getrennt nebeneinander gestellt sind. Die kommerziell erhältlichen Polyethylenglykole sind beispielsweise unter den Handelsnamen Carbowax^{®} (Union Carbide), Emkapol^{®} und Renex^{®} PEG (ICI), Lipoxol^{®} (DEA), Polyglykol^{®} E (Dow), Pluracol^{®} E, Pluriol^{®} E sowie Lutrol^{®} E (BASF) verfügbar.

Polypropylenglykole (PPG) sind über einen breiten Molmassenbereich von 250 (PPG-4) bis 4.000 (PPG-69) klare, nahezu farblose Flüssigkeiten, für deren Bezeichnung die zuvor beschriebene INCI-Nomenklatur analog verwendet wird. So werden die Polypropylenglykole der obigen allgemeinen Formel mit Werten n von 5 bzw. 6 als PEG-5 bzw. PEG-6 bezeichnet. Die niedermolekularen Polypropylenglykole sind mit Wasser mischbar, während die höhermolekularen Vertreter weniger wasserlöslich sind. Kommerziell erhältlich sind beispielsweise die gemäß INCI bezeichneten Polypropylenglykole PPG-7, PPG-9, PPG-12, PPG-13, PPG-15, PPG-17, PPG-20, PPG-26, PPG-30, PPG-33, PPG-34, PPG-51 und PPG-69. Bezugsquellen sind dem International Cosmetic Ingredient Dictionary and Handbook zu entnehmen.

Bei den Copolymeren handelt es sich vorzugsweise um statistische Copolymere und insbesondere um Blockcopolymere aus Ethylen- und Propylenoxid, Ethylenoxid und Tetrahydrofuran, Propylenoxid und Tetrahydrofuran oder Ethylenoxid, Propylenoxid und Tetrahydrofuran, bevorzugt aus Ethylen- und Propylenoxid, besonders bevorzugt um Blockcopolymere aus Ethylen- und Propylenoxid.

Erfindungsgemäß bevorzugte statistische Copolymere aus a Ethylen- und b Propylenoxideinheiten sind beispielsweise folgende gemäß International Cosmetic Ingredient Dictionary and Handbook als PEG/PPG-a/b bezeichnete Copolymere (Molmasse), wobei a und b Mittelwerte darstellen: PEG/PPG-18/4 Copolymer (1000), PEG/PPG-17/6 Copolymer (1100), PEG/PPG-35/9 Copolymer (2100) und PEG/PPG-23/50 Copolymer (3900).

Erfindungsgemäß bevorzugte Blockcopolymere aus Ethylen- und Propylenoxid genügen der Formel HO(CH₂CH₂O)ₓ(CH(CH₃)CH₂O)_{y}(CH₂CH₂O)_{x'}H in der x und x' für Mittelwerte von 2 bis 130 und y für Mittelwerte von 15 bis 67 stehen, und werden mit dem internationalen Freinamen Poloxamer bezeichnet, der auch im International Cosmetic Ingredient Dictionary and Handbook verwendet wird. Jedes Poloxamer ist durch eine dreistellige Nummer gekennzeichnet. Die ersten beiden Ziffern geben multipliziert mit 100 die durchschnittliche Molmasse des Polypropylenglykol-Anteils und die letzte Ziffer multipliziert mit 10 den Polyethylenglykol-Anteil in Gew.-% an. Dieser beträgt 10 bis 80 Gew.- %, vorzugsweise nicht mehr als 50 Gew.-%, insbesondere nicht mehr als 40 Gew.-%, besonders bevorzugt nicht mehr als 30 Gew.-%, beispielsweise 10, 20 oder 30 Gew.-%. Die Herstellung der Poloxamere erfolgt zweistufig, wobei zunächst Propylenoxid kontrolliert an Propylenglykol addiert und der erhaltene Polypropylenglykolblock durch anschließende Addition von Ethylenoxid von zwei Polyethylenglykol-Blöcken eingefasst wird. Besonders bevorzugte Blockcopolymere sind beispielsweise folgende flüssige Poloxamer-Typen (x, y, x'; Molmasse; z. T. Schmelzpunkt): Poloxamer 101 (2, 16, 2; 1100; -32), Poloxamer 122 (5, 21, 5; 1630; -26), Poloxamer 123 (7, 21, 7; 1900; -1), Poloxamer 105 (11, 16, 11; 1850; 7), Poloxamer 181 (3, 30, 3; 2000; -29), Poloxamer 124 (11, 21, 11; 2200; 16), Poloxamer 182 (8, 30, 8; 2500; -4), Poloxamer 183 (10, 30, 10; 2650; 10), Poloxamer 212 (8, 35, 8; 2750; -7), Poloxamer 231 (6, 39, 6; 2750; -37), Poloxamer 184 (13, 30, 13; 2900; 16), Poloxamer 185 (19, 30, 19; 3400), Poloxamer 282 (10, 47, 10; 3650; 7), Poloxamer 331 (7, 54, 7; 3800; -23), Poloxamer 234 (22, 39, 22; 4200; 18), Poloxamer 401 (6, 67, 6; 4400; 5), Poloxamer 284 (21, 47, 21; 4600) und Poloxamer 402 (13, 67, 13; 5000; 20). Kommerziell sind die Poloxamere unter den Handelsnamen Pluronic^{®} und Synperonic^{®} PE erhältlich, den ein Buchstabe aus der Gruppe L, P und F sowie eine zwei- oder dreistellige Zahl nachgestellt ist. Dabei ist die letzte Ziffer mit der letzten Ziffer der Poloxamer-Nomenklatur identisch und ergeben die davorstehenden ein- oder zweistelligen Zahlen multipliziert mit 300 die ungefähre Molmasse des Polypropylenglykol-Anteils bzw. mit 3 multipliziert in etwa die aus den ersten beiden Ziffern der Poloxamer-Nomenklatur-Nummer gebildeten Zahl, d. h. entsprechen 3, 4, 6, 7, 8, 9, 10 und 12 in dieser Reihenfolge den zweistelligen Zahlen 10, 12, 18, 21, 23, 28, 33 und 40 am Anfang der Nummer gemäß der Poloxamer-Nomenklatur. Die Buchstaben unterscheiden flüssige (L), pastöse (P) und feste (F) Poloxamere. So ist beispielsweise das Poloxamer 101 als Pluronic^{®} L 31 und Synperonic^{®} PE L 31 erhältlich.

Eine weitere Klasse geeigneter Blockcopolymere aus Ethylen- und Propylenoxid genügen der Formel HO(CH(CH₃)CH₂O)_{y}(CH₂CH₂O)ₓ(CH₂CH(CH3)O)_{y'}H. Hier wird ein Polyethylenglykol-Block von zwei Polypropylenglykol-Blöcken eingerahmt, während bei den Poloxameren ein Polypropylenglykol-Block von zwei Polyethylenglykol-Blöcken eingefasst wird. Die Herstellung erfolgt wiederum zweistufig, wobei zunächst Ethylenoxid kontrolliert an Ethylenglykol addiert und der erhaltene Polyethylenglykol-Block durch anschließende Addition von Propylenoxid von zwei Polypropylenglykol-Blöcken eingefasst wird. Kommerziell erhältlich sind diese Blockcopolymere wie die Poloxamere unter dem Handelsnamen Pluronic^{®} (BASF), dem jeweils ein alphanumerischer Code aus drei Ziffern und dem zwischen die zweite und dritte Ziffer geschobenen Buchstaben R folgt. Die Bedeutung der Ziffern ist mit der Bedeutung im Rahmen der Poloxamer-Nomenklatur identisch. Der eingeschobene Buchstabe R (für engl. reverse) deutet auf die im Vergleich zu den Poloxameren invertierte Struktur hin. Bevorzugte Vertreter dieser Klasse sind die folgenden Pluronic^{®}-Typen (Molmasse; Schmelzpunkt): Pluronic^{®} 10R5 (1950; 15), Pluronic^{®} 12R3 (1800; - 20), Pluronic^{®} 17R1 (1900; -27), Pluronic^{®} 17R2 (2150; -25), Pluronic^{®} 17R4 (2650; 18), Pluronic^{®} 25R1 (2700; -5), Pluronic^{®} 25R2 (3100; - 5), Pluronic^{®} 31R1 (3250; -25) und Pluronic^{®} 31R2 (3300; 9).

In einer weiteren bevorzugten Ausführungsform können eine oder mehrere der endständigen Hydroxygruppen der zuvor genannten Alkylenglykole zusätzlich verethert sein. In den bevorzugten endständigen veretherten Alkylenglykolen tragen sind die Wasserstoffatome der Hydroxygruppen durch lineare oder verzweigte, gesättigte oder ungesättigte Alkylgruppen mit 1 bis 30 Kohlenstoffatomen ersetzt.

Die Polyalkylenglykole werden vorteilhafter Weise in einer Menge von 0,001 bis 8 Gewichtsprozent, vorzugsweise 0,01 bis 5 Gewichtsprozent, besonders bevorzugt von 0,01 bis 3 Gewichtsprozent und insbesondere von 0,01 bis 1,5 Gewichtsprozent, jeweils bezogen auf das Gesamtgewicht des polymeren Substrats und der Zusammensetzung, eingesetzt.

Vorzugsweise ist das Gewichtsverhältnis von polymerem Substrat zu Zusammensetzung 1:5 bis 1:30, vorzugsweise 1:10 bis 1:20 für den Fall, dass die Zusammensetzung mit einem polymeren Substrat in Kontakt gebracht wird, d.h. auf das Substrat aufgebracht wird.

Für den Fall, dass die Zusammensetzung während der Polymerisation des polymeren Substrats zugegen ist, liegt das Gewichtsverhältnis von Prepolymermischung zu Zusammensetzung bevorzugt bei 80:1 bis 0.1:1 weiter bevorzugt bei 50:1 bis 0.1:1.

Ein weiterer Gegenstand der vorliegenden Erfindung ist die Verwendung der erfindungsgemäßen Wundauflage zur Behandlung von Wunden, insbesondere Wunden, die anfällig für mikrobiologische Verunreinigung sind oder die mikrobiologisch verunreinigt oder infiziert sind, im Speziellen Brandwunden, Diabetes und Dekubitus ulcus.

Die erfindungsgemäße Wundauflage kann in einer bevorzugten Ausgestaltung auch mehrlagig ausgestaltet sein. Bevorzugt ist es beispielsweise, dass das polymere Substrat der erfindungsgemäßen Wundauflage einseitig mit einem Adhäsiv beschichtet ist, dass einen guten Wundkontakt sicherstellt, aber ein Verkleben der Wundauflage mit der Wunde insbesondere bei geringer Aufnahme von Wundexsudat verhindert. Diese Adhäsiv-Schicht besteht bevorzugt aus Silikon. Eine andere bevorzugte Ausführungsform besteht darin, dass die Wundauflage an der von der Wunde abgewandten Seite mit einer feuchtigkeitsundurchlässigen Schicht, beispielsweise einem Polymerfilm abgedeckt ist, um die Handhabung der Wundauflage zu vereinfachen. Dabei kann dieser Polymerfilm vorzugsweise bestehen aus Silikon, Polyamid oder einem wasserundurchlässigen Polyurethan.

Ein weiterer Gegenstand der vorliegenden Erfindung ist ein Verfahren zur Herstellung einer Wundauflage, wobei ein polymeres Substrat durch Polymerisationsreaktion in Gegenwart einer Zusammensetzung umfassend
a) mindestens einen antimikrobiellen Wirkstoff und
b) ein die Zytotoxizität absenkendes Mittel, umfassend eine Öl in Wasser Emulsion, die zusätzlich eine oder mehrere Alkandiole mit 3 bis 12 Kohlenstoffatomen aufweist,
hergestellt wird.

In einer besonders bevorzugten Ausführungsform handelt es sich bei der Polymerisationsreaktion zur Herstellung des polymeren Substrats um eine Reaktion eines Polyisocyanats mit einem Polyol.

Ein weiterer Gegenstand der vorliegenden Erfindung ist ein Verfahren zur Herstellung einer Wundauflage gemäß der vorliegenden Erfindung, umfassend die folgenden Schritte:
a) Bereitstellen einer Prepolymermischung für die Herstellung eines Polyurethanschaums
b) Bereitstellen einer wässrigen Zusammensetzung, umfassend
   (1) mindestens einen antimikrobiellen Wirkstoff und
   (2) ein die Zytotoxizität absenkendes Mittel, umfassend eine Öl in Wasser Emulsion, die zusätzlich eine oder mehrere Alkandiole mit 3 bis 12 Kohlenstoffatomen aufweist und
c) Vereinigen der Pre-Polymermischung und der wässrigen Zusammensetzung unter Ausbildung eines Polyurethanschaums und
d) gegebenenfalls Sterilisieren, vorzugsweise durch Gamma-Strahlen oder mittels Dampfdrucksterilisation.

Geeignete und bevorzugte Prepolymere, die für das erfindungsgemäße Verfahren eingesetzt werden können, wurden bereits zuvor beschrieben. Ein weiterer Gegenstand der vorliegenden Erfindung ist ein Verfahren zur Herstellung einer erfindungsgemäßen Wundauflage, umfassend die folgenden Schritte:
a) Bereitstellen eines Flüssigkeits-absorbierenden polymeren Substrats, und
b)in Kontakt bringen des Substrats mit einer wässrigen Zusammensetzung, umfassend
   (1) mindestens einen antimikrobiellen Wirkstoff und
   (2) ein die Zytotoxizität absenkendes Mittel, umfassend eine Öl in Wasser Emulsion, die zusätzlich eine oder mehrere Alkandiole mit 3 bis 12 Kohlenstoffatomen aufweist und
c) gegebenenfalls Trocknen und
d) gegebenenfalls Sterilisieren, vorzugsweise durch Gamma-Strahlen oder
mittels Dampfdrucksterilisation.

Geeignete und bevorzugte Komponenten, die in den Zusammensetzungen enthalten sein können, die in den erfindungsgemäßen Verfahren eingesetzt werden, sind bereits zuvor im Zusammenhang mit der erfindungsgemäßen Wundauflage beschrieben worden.

### Beispiele

Es wurde ein Flüssigkeit absorbierender, hydrophiler Polyurethanschaumstoff auf Basis eines mit Toluoldiisocyanat verkappten Prepolymers hergestellt. Die für die Herstellung des Schaums verwendeten Zusammensetzungen sind in den Tabellen 1 und 2 wiedergegeben.

Die Prepolymermischung kann mit den in den Tabellen 1 und 2 beschriebenen Zusammensetzungen in einem geeigneten Verhältnis vereinigt und die Mischung aufgeschäumt werden, so dass eine gewünschte Endkonzentration des antimikrobiellen Wirkstoffs PHMB erzielt wird.

In allen Beispielen ist zu berücksichtigen, dass herstellungsbedingt ein Teil des Wassers verdunsten kann.

Die in den nachfolgenden Tabellen 1 und 2 dargestellten Zusammensetzungen zeigen geeignete Zusammensetzungen, die auf ein polymeres Substrat für die erfindungsgemäßen Wundauflagen aufgebracht werden können oder zusammen mit einem geeigneten Prepolymer zu einem Schaum verarbeitet werden können.

**Tabelle 1**

| Erfindungsgemäß einzusetzende Öl in Wasser Emulsion mit Alkandiol | |
|---|---|
| Menge in Gewichtsprozent (Gew.-%) | Komponente |
| 10 Gew.-% | mittelkettiges Triglycerid¹⁾ |
| 10 Gew.-% | Sojabohnenöl |
| 2,25 Gew.-% | 1,2-Octandiol |
| 1,2 Gew.-% | Eilecithin |
| 0,25 Gew.-% | Natriumoleat |
| 0,2 Gew.-% | α-Tocopherol |
| 1,5 Gew.-% | PHMB (20 %) ²⁾ |
| ad 100 | Wasser |

| | |
|---|---|
| ¹⁾ Glycerin, welches mit Fettsäuren verestert ist, die bis zu 98 mol.-% aus Caprylsäure (C₈) und Caprinsäure (C₁₀) bestehen ²⁾ wässrige Lösung enthaltend 20 Gew.-% Polyhexamethylenbiguanid Hydrochlorid (PHMB) | |

**Tabelle 2**

| Erfindungsgemäß einzusetzende Öl in Wasser Emulsion mit Alkandiol | |
|---|---|
| Menge in Gewichtsprozent (Gew.-%) | Komponente |
| 20 Gew.-% | Triisostearin |
| 2,25 Gew.-% | 1,2-Octandiol |
| 1,2 Gew.-% | Hydriertes Sojalecithin |
| 0,25 Gew.-% | Natriumisostearat |
| 1,5 Gew.-% | PHMB (20 %) ¹⁾ |
| ad 100 | Wasser |

| | |
|---|---|
| ¹⁾ wässrige Lösung enthaltend 20 Gew.-% Polyhexamethylenbiguanid Hydrochlorid (PHMB) | |

Es hat sich überraschend gezeigt, dass der Einsatz aliphatischer Alkandiole, wie beispielsweise das in den Tabellen 1 und 2 eingesetzte 1,2-Octandiol eine Reduzierung des einzusetzenden antimikrobiellen Wirkstoffs bei gleichem antimikrobiellem Effekt ermöglicht. Dies verringert weiterhin die Zytotoxizität, da für den gleichen antimikrobiellen Effekt eine niedrigere Konzentration des antimikrobiellen Wirkstoffs benötigt wird.

## Patentansprüche

1. Wundauflage umfassend ein polymeres Substrat und eine Zusammensetzung umfassend
a) mindestens einen antimikrobiellen Wirkstoff und
b) ein die Zytotoxizität absenkendes Mittel, umfassend eine Öl in Wasser Emulsion, die zusätzlich ein oder mehrere Alkandiol(e) mit 3 bis 12 Kohlenstoffatomen aufweist.

2. Wundauflage gemäß Anspruch 1, **dadurch gekennzeichnet, dass** das polymere Substrat Flüssigkeit absorbieren kann und als zellulärer Schaum vorliegt.

3. Wundauflage gemäß mindestens einem der Ansprüche 1 bis 2, **dadurch gekennzeichnet, dass** das polymere Substrat ausgewählt ist aus der Gruppe bestehend aus Polyethylenterephthalat, Polyethylen, Polypropylen, Rayon, Baumwolle, Nylon-6,6, Polycaprolactam, Polyurethan sowie beliebige Mischungen und Kombinationen hiervon.

4. Wundauflage gemäß mindestens einem der Ansprüche 1 bis 3, **dadurch gekennzeichnet, dass** das polymere Substrat Polyurethan, vorzugsweise ein bei einer Temperatur von 20 bis 45°C aufgeschäumtes Polyurethan oder ein thermoplastisches Polyurethan ist.

5. Wundauflage gemäß mindestens einem der Ansprüche 1 bis 4, **dadurch gekennzeichnet, dass** der antimikrobielle Wirkstoff ausgewählt ist aus der Gruppe bestehend aus Polymethylenbiguanid, Polyhexamethylenbiguanid, Polyhexamethylenguanid, Chlorhexidin, Hexetidin, Iodophoren, PVP-Iod, 2,4,4'-Trichlor-2-hydroxydiphenylether, Octenidin, sowie deren Salze, vorzugsweise deren Hydrohalogenide, Acetate, Gluconate oder Sulfate, insbesondere Chlorhexidinacetat, Chlorhexidingluconat, Chlorhexidinsulfat, Chlorhexidinhydrochlorid, Polyhexamethylenbiguanid Hydrochlorid und Octenidinhydrochlorid.

6. Wundauflage gemäß mindestens einem der Ansprüche 1 bis 5, **dadurch gekennzeichnet, dass** der antimikrobielle Wirkstoff erhältlich ist durch Polykondensation eines Guanidinsäureadditionssalzes mit einem Amingemisch, welches wenigstens ein Diamin und/oder ein Triamin enthält, wobei vorzugsweise wenigstens ein Amin ausgewählt ist aus der Gruppe, die aus
i) Diamin, das wenigstens einen cycloaliphatischen Rest aufweist, und
ii) Dialkylentriamin besteht.

7. Wundauflage gemäß mindestens einem der Ansprüche 1 bis 6, **dadurch gekennzeichnet, dass** der antimikrobielle Wirkstoff in einer Menge von bis zu maximal 10 Gew.-%, insbesondere 0,01 bis 5 Gew.-% und im Speziellen in einer Menge von 0,1 bis 4 Gew.-%, bezogen auf das Gesamtgewicht des Polymersubstrats und der Zusammensetzung, vorliegt.

8. Wundauflage gemäß mindestens einem der Ansprüche 1 bis 7, **dadurch gekennzeichnet, dass** die Öl in Wasser Emulsion mindestens ein Phospholipid, das vorzugsweise Lecithin ist, welches besonders bevorzugt aus Sojabohnen und/oder Eiern stammt, und mindestens eine Ölkomponente, ausgewählt aus mittelkettigen Triglyceriden, Sojabohnenöl, Saffloröl, Isostearin oder Mischungen hiervon, ist.

9. Wundauflage gemäß mindestens einem der vorangehenden Ansprüche, **dadurch gekennzeichnet, dass** die Zusammensetzung Polyalkylenglykole mit einer mittleren Molmasse zwischen 200 und 10000, vorzugsweise 500 bis 8000 und weiter bevorzugt 1000 bis 6000, insbesondere 1500 bis 5000 Dalton aufweist.

10. Wundauflage gemäß mindestens einem der vorangehenden Ansprüche, **dadurch gekennzeichnet, dass** die Zusammensetzung Alkandiole mit 3 bis 12 Kohlenstoffatomen aufweist, ausgewählt aus der Gruppe bestehend aus 1,2 Propylenglykol, 1,3-Butylenglykol, 1,2-Pentandiol, 1,2-Hexandiol, 1,6-Hexandiol und/oder 1,2-Octandiol.

11. Wundauflage gemäß Anspruch 10, **dadurch gekennzeichnet, dass** das Alkandiol 1,2-Octandiol ist.

12. Wundauflage gemäß mindestens einem der vorangehenden Ansprüche zur Verwendung bei der Behandlung von Wunden, insbesondere Wunden, die anfällig für mikrobiologische Verunreinigungen oder die mikrobiologisch verunreinigt oder infiziert sind, im Speziellen Brandwunden, Diabetes und Dekubitus ulcus.

13. Verfahren zur Herstellung einer Wundauflage, **dadurch gekennzeichnet, dass** ein polymeres Substrat durch Polymerisationsreaktion in Gegenwart einer Zusammensetzung umfassend
a) mindestens einen antimikrobiellen Wirkstoff und
b) ein die Zytotoxizität absenkendes Mittel, umfassend eine Öl in Wasser Emulsion, die zusätzlich eine oder mehrere Alkandiole mit 3 bis 12 Kohlenstoffatomen aufweist,
hergestellt wird.

14. Verfahren zur Herstellung einer Wundauflage gemäß mindestens einem der Ansprüche 1 bis 12, umfassend die folgenden Schritte:
a) Bereitstellen einer Prepolymermischung für die Herstellung eines Polyurethanschaums
b) Bereitstellen einer wässrigen Zusammensetzung, umfassend
(1) mindestens einen antimikrobiellen Wirkstoff und
(2) ein die Zytotoxizität absenkendes Mittel, umfassend eine Öl in Wasser Emulsion, die zusätzlich eine oder mehrere Alkandiole mit 3 bis 12 Kohlenstoffatomen aufweist und
c) Vereinigen der Pre-Polymermischung und der wässrigen Zusammensetzung unter Ausbildung eines Polyurethanschaums und
d) gegebenenfalls Sterilisieren, vorzugsweise durch Gamma-Strahlen oder mittels Dampfdrucksterilisation.

15. Verfahren zur Herstellung einer Wundauflage gemäß mindestens einem der Ansprüche 1 bis 12, umfassend die folgenden Schritte:
a) Bereitstellen eines Flüssigkeits-absorbierenden polymeren Substrats, und
b) in Kontakt bringen des Substrats mit einer wässrigen Zusammensetzung, umfassend
(1) mindestens einen antimikrobiellen Wirkstoff und
(2) ein die Zytotoxizität absenkendes Mittel, umfassend eine Öl in Wasser Emulsion, die zusätzlich eine oder mehrere Alkandiole mit 3 bis 12 Kohlenstoffatomen aufweist und
c) gegebenenfalls Trocknen und
d) gegebenenfalls Sterilisieren, vorzugsweise durch Gamma-Strahlen oder mittels Dampfdrucksterilisation.

## Claims

1. A wound dressing comprising a polymeric substrate and a composition comprising
a) at least one antimicrobially active substance; and
b) a cytotoxicity-reducing agent comprising an oil-in-water emulsion that additionally contains one or more alkanediols having from 3 to 12 carbon atoms.

2. The wound dressing according to claim 1, **characterized in that** said polymeric substrate can absorb liquid, and is a cellular foam.

3. The wound dressing according to at least one of claims 1 to 2, **characterized in that** said polymeric substrate is selected from the group consisting of polyethylene terephthalate, polyethylene, polypropylene, rayon, cotton, nylon-6,6, polycaprolactam, polyurethane as well as any mixtures and combinations thereof.

4. The wound dressing according to at least one of claims 1 to 3, **characterized in that** said polymeric substrate is polyurethane, preferably a polyurethane foamed at a temperature of from 20 to 45 °C, or a thermoplastic polyurethane.

5. The wound dressing according to at least one of claims 1 to 4, **characterized in that** said antimicrobially active substance is selected from the group consisting of polymethylene biguanide, polyhexamethylene biguanide, polyhexamethylene guanide, chlorhexidine, hexetidine, iodophors, PVP-iodine, 2,4,4'-trichloro-2-hydroxydiphenyl ether, octenidine, and salts thereof, preferably hydrohalides, acetates, gluconates or sulfates thereof, especially chlorhexidine acetate, chlorhexidine gluconate, chlorhexidine sulfate, chlorhexidine hydrochloride, polyhexamethylene biguanide hydrochloride and octenidine hydrochloride.

6. The wound dressing according to at least one of claims 1 to 5, **characterized in that** said antimicrobially active substance is obtainable by the polycondensation of a guanidine acid addition salt with a mixture of amines which contains at least one diamine and/or triamine, wherein preferably at least one amine is selected from the group consisting of
i) a diamine having at least one cycloaliphatic radical; and
ii) a dialkylene triamine.

7. The wound dressing according to at least one of claims 1 to 6, **characterized in that** said antimicrobially active substance is present in an amount of up to a maximum of 10% by weight, particularly from 0.01 to 5% by weight, and especially in an amount of from 0.1 to 4% by weight, based on the total weight of the polymeric substrate and the composition.

8. The wound dressing according to at least one of claims 1 to 7, **characterized in that** said oil-in-water emulsion is at least one phospholipid, which is preferably a lecithin, which is more preferably derived from soybeans and/or eggs, and at least one oil component selected from medium-chain triglycerides, soybean oil, safflower oil, isostearin, or mixtures thereof.

9. The wound dressing according to at least one of the preceding claims, **characterized in that** said composition contains polyalkylene glycols with an average molecular weight of from 200 to 10,000, preferably from 500 to 8,000, more preferably from 1000 to 6000, especially from 1500 to 5000, daltons.

10. The wound dressing according to at least one of the preceding claims, **characterized in that** said composition contains alkanediols having from 3 to 12 carbon atoms, selected from the group consisting of 1,2-propylene glycol, 1,3-butylene glycol, 1,2-pentanediol, 1,2-hexanediol, 1,6-hexanediol and/or 1,2-octanediol.

11. The wound dressing according to claim 10, **characterized in that** said alkanediol is 1,2-octanediol.

12. The wound dressing according to at least one of the preceding claims for use in the treatment of wounds, especially wounds that are susceptible to microbiological contamination, or that are microbiologically contaminated or infected, especially burn injuries, diabetes and decubitus ulcer.

13. A process for preparing a wound dressing, **characterized in that** a polymeric substrate is prepared by a polymerization reaction in the presence of a composition comprising
a) at least one antimicrobially active substance; and
b) a cytotoxicity-reducing agent comprising an oil-in-water emulsion that additionally contains one or more alkanediols having from 3 to 12 carbon atoms.

14. A process for preparing a wound dressing according to at least one of claims 1 to 12, comprising the following steps:
a) providing a prepolymer mix for the preparation of a polyurethane foam;
b) providing an aqueous composition comprising
1) at least one antimicrobially active substance; and
2) a cytotoxicity-reducing agent comprising an oil-in-water emulsion that additionally contains one or more alkanediols having from 3 to 12 carbon atoms; and
c) combining the prepolymer mix and the aqueous composition to form a polyurethane foam; and
d) optionally sterilizing, preferably by gamma rays or by means of highpressure steam sterilization.

15. A process for preparing a wound dressing according to at least one of claims 1 to 12, comprising the following steps:
a) providing a liquid-absorbing polymeric substrate; and
b) contacting the substrate with an aqueous composition comprising
1) at least one antimicrobially active substance; and
2) a cytotoxicity-reducing agent comprising an oil-in-water emulsion that additionally contains one or more alkanediols having from 3 to 12 carbon atoms; and
c) optionally drying; and
d) optionally sterilizing, preferably by gamma rays or by means of highpressure steam sterilization.

## Revendications

1. Pansement comprenant un substrat polymère et une composition comprenant
a) au moins un principe actif antimicrobien, et
b) un agent réduisant la cytotoxicité, comprenant une émulsion huile dans eau présentant en outre un ou plusieurs alcanediols avec 3 à 12 atomes de carbone.

2. Pansement selon la revendication 1, **caractérisé en ce que** le substrat polymère peut absorber du liquide et se présente sous forme de mousse cellulaire.

3. Pansement selon au moins une des revendications 1 à 2, **caractérisé en ce que** le substrat polymère est choisi dans le groupe consistant en polytéréphtalate d'éthylène, polyéthylène, polypropylène, rayon, coton, nylon-6,6, polycaprolactame, polyuréthane et tout mélange et toute combinaison de ceux-ci.

4. Pansement selon au moins une des revendications 1 à 3, **caractérisé en ce que** le substrat polymère est un polyuréthane, de préférence un polyuréthane moussé à une température de 20 à 45 °C, ou un polyuréthane thermoplastique.

5. Pansement selon au moins une des revendications 1 à 4, **caractérisé en ce que** le principe actif antimicrobien est choisi dans le groupe consistant en biguanide de polyméthylène, biguanide de polyhexaméthylène, guanide de polyhexaméthylène, chlorhexidine, hexétidine, iodophores, povidone iodée, l'éther 2,4,4'-trichloro-2-hydroxydiphénylique, octénidine et des sels de ceux-ci, de préférence des hydrohalogénures, acétates, gluconates ou sulfates de ceux-ci, notamment l'acétate de chlorhexidine, le gluconate de chlorhexidine, le sulfate de chlorhexidine, l'hydrochlorure de chlorhexidine, et l'hydrochlorure d'octénidine.

6. Pansement selon au moins une des revendications 1 à 5, **caractérisé en ce que** le principe actif antimicrobien peut être obtenu par la polycondensation d'un sel d'addition d'acide de guanidine avec un mélange d'amines contenant au moins une diamine et/ou une triamine, de préférence au moins une amine étant choisie dans le groupe consistant en
i) une diamine ayant au moins un reste cycloaliphatique, et
ii) une dialkylènetriamine.

7. Pansement selon au moins une des revendications 1 à 6, **caractérisé en ce que** le principe actif antimicrobien se présente en une quantité de jusqu'à 10 % en poids au maximum, notamment de 0,01 à 5 % en poids, en particulier en une quantité de 0,1 à 4 % en poids, par rapport au poids total du substrat polymère et de la composition.

8. Pansement selon au moins une des revendications 1 à 7, **caractérisé en ce que** ladite émulsion huile dans eau consiste en au moins un phospholipide, de préférence de la lécithine, de préférence encore issue de fèves de soja et/ou d'oeufs, et au moins un composant huileux choisi parmi les triglycérides à chaîne moyenne, l'huile de soja, l'huile de carthame, l'isostéarine, ou des mélanges de ceux-ci.

9. Pansement selon au moins une des revendications précédentes, **caractérisé en ce que** la composition contient des polyalkylèneglycols avec une masse moléculaire moyenne entre 200 et 10000, de préférence 500 à 8000, et de préférence encore 1000 à 6000, notamment 1500 à 5000 daltons.

10. Pansement selon au moins une des revendications précédentes, **caractérisé en ce que** la composition contient des alcanediols avec 3 à 12 atomes de carbone, choisis dans le groupe consistant en 1,2-propylèneglycol, 1,3-butylèneglycol, 1,2-pentanediol, 1,2-hexanediol, 1,6-hexanediol et/ou 1,2-octanediol.

11. Pansement selon la revendication 10, **caractérisé en ce que** ledit alcanediol est le 1,2-octanediol.

12. Pansement selon au moins une des revendications précédentes à utiliser dans le traitement de plaies, notamment de plaies vulnérables à des contaminations microbiologiques, ou contaminées ou infectées microbiologiquement, notamment des brûlures, le diabète, et décubitus.

13. Procédé pour la production d'un pansement, **caractérisé en ce que** l'on prépare un substrat polymère par une réaction de polymérisation en présence d'une composition comprenant
a) au moins un principe actif antimicrobien, et
b) un agent réduisant la cytotoxicité, comprenant une émulsion huile dans eau présentant en outre un ou plusieurs alcanediols avec 3 à 12 atomes de carbone.

14. Procédé pour la production d'un pansement selon au moins une des revendications 1 à 12, comprenant les étapes consistant à :
a) procurer un mélange de prépolymères pour la production d'une mousse de polyuréthane,
b) procurer une composition aqueuse comprenant
1) au moins un principe actif antimicrobien, et
2) un agent réduisant la cytotoxicité, comprenant une émulsion huile dans eau présentant en outre un ou plusieurs alcanediols avec 3 à 12 atomes de carbone, et
c) mélanger le mélange de prépolymères et la composition aqueuse pour former une mousse de polyuréthane, et
d) éventuellement stériliser, de préférence au moyen de rayonnements gamma ou de stérilisation sous pression de vapeur.

15. Procédé pour la production d'un pansement selon au moins une des revendications 1 à 12, comprenant les étapes consistant à :
a) procurer un substrat polymère pouvant absorber du liquide, et
b) mettre le substrat en contact avec une composition aqueuse comprenant
1) au moins un principe actif antimicrobien, et
2) un agent réduisant la cytotoxicité, comprenant une émulsion huile dans eau présentant en outre un ou plusieurs alcanediols avec 3 à 12 atomes de carbone, et
c) éventuellement sécher, et
d) éventuellement stériliser, de préférence au moyen de rayonnements gamma ou de stérilisation sous pression de vapeur.
